# EUROPEAN PATENT SPECIFICATION

(11) **EP 4 366 615 B1**
(45) Date of publication and mention of the grant of the patent: **02.12.2026**
(21) Application number: 22744731.5
(22) Date of filing: 07.07.2022
(51) Int. Cl.: A61B 5/11, A61B 5/00

(54) **FALL RISK DETERMINATION SYSTEM AND METHOD**
SYSTEM UND VERFAHREN ZUR BESTIMMUNG DES STURZRISIKOS
SYSTÈME ET PROCÉDÉ DE DÉTERMINATION DE RISQUE DE CHUTE

(30) Priority: 09.07.2021 EP 21184820
(43) Date of publication of application: 15.05.2024
(73) Proprietor: Infonomy AB, 223 81 Lund (SE)
(72) Inventor: BJERKBORN, Simon, 224 76 Lund (SE); KRISTEN, Helmuth, 241 31 Eslöv (SE); KÄLLMÉN, Jonas, 216 18 Limhamn (SE)
(74) Representative: Brann AB
(86) International application number: PCT/EP2022/068895
(87) International publication number: WO 2023/280974

(56) References cited:
- EP-B1- 3 649 939
- WO-A1-2021/233725
- US-A1- 2010 152 623
- US-A1- 2012 004 881
- US-A1- 2012 119 904
- US-A1- 2013 151 193
- US-A1- 2016 100 776
- US-A1- 2021 097 840
- US-B2- 11 039 760
- BRODIE MATTHEW A ET AL: "Eight-Week Remote Monitoring Using a Freely Worn Device Reveals Unstable Gait Patterns in Older Fallers", IEEE TRANSACTIONS ON BIOMEDICAL ENGINEERING, IEEE, USA, vol. 62, no. 11, 1 November 2015 (2015-11-01), pages 2588 - 2594, XP011587176, ISSN: 0018-9294, [retrieved on 20151020], DOI: 10.1109/TBME.2015.2433935

## Description

### TECHNICAL FIELD

Embodiments herein relate to a system, a corresponding method, computer program and carrier for a computer program, for determining, for a bipedal object being a human being, a fall risk measure that represents a risk for the bipedal object to fall when walking.

### BACKGROUND

One of the most common reasons for immobilization, injury and even death among elderly people is falls when walking. It is therefore very important to address the issue of preventing falls, not least when also considering that the number of people of high age will increase in the future due to the fact that the average life expectancy is increasing in most populations.

It has been found that gait disorder is one of the most relevant factors when it comes to determining the risk for falling when walking. In order to determine gait disorder it is necessary to monitor individuals over longer periods of time and during such monitoring estimate and record gait parameters. Having recorded gait parameters over a period of time and during various walking situations, e.g. during indoor walking on a flat floor surface, outdoor walking on non-flat surfaces and during walking up and down stairs etc., the gait parameters are processed and thereby yielding some kind of measure of a person's risk of falling when walking. In other words, determination of gait disorder is a procedure that typically requires long periods of observation and typically requires observational skills of trained persons followed by subsequent processing of the gait parameters. Such a procedure is therefore typically very time consuming and therefore very expensive.

BRODIE MATTHEW A ET AL: "Eight-Week Remote Monitoring Using a Freely Worn Device Reveals Unstable Gait Patterns in Older Fallers", IEEE TRANSACTIONS ON BIOMEDICAL ENGINEERING, IEEE, vol. 62, no. 11, 1 November 2015 (2015-11-01), pages 2588-2594, discloses algorithms to detect gait impairments remotely using data from freely worn devices during long term monitoring and statistical models that describe how gait performances are distributed over several weeks.

Less expensive ways may possibly be considered when noting that current digital technology includes devices such as step counters and various mobile units equipped with accelerometers. However, such devices, i.e. mobile smart phones and the like, are typically configured for recording various exercise sessions, most of which are focused on leisure activities of a younger part of the population; they are not configured to perform the much needed task of determining gait disorder of elderly persons and to provide a reliable estimate of a risk of falling

### SUMMARY

In view of the above, an object of the present disclosure is to overcome drawbacks related to prior art methods and arrangements for determining the risk of falling.

Such an object is achieved in one aspect by a system according to claim 1.

In other words, such a system is configured with circuitry that provides a determination of a fall risk measure in a continuous real-time manner, which is in contrast to prior art procedures that require long periods of observation and observational skills of trained persons followed by subsequent data processing in order to obtain a fall risk measure. Use of the system of the present disclosure is therefore less time consuming and less expensive to operate than prior art systems and procedures while at the same time providing a high-quality fall risk measure.

The term "bipedal object" is understood as an object having two legs. Specifically, the bipedal object has a structure having functions of a knee joint in each leg. This means the structure is a hinge joint, which permits flexion and extension, and thereby propelling, of the leg. The function allows for walking which is defined as one foot is moved forward in front of another. The term "walking" is understood as taking a plurality of steps. The forward motion of the walk occurs when the raised leg is propelled forward.

It is understood that the term "robot" encompasses anthrobot which is a robot that is either entirely or in some way human-like. Specifically, the term "robot" is understood as a humanoid robot, which is a robot resembling the human body in shape. More specifically, the term "robot" relates to a robot having a knee joint as described above in each of the legs.

The system may comprise an accelerometer, wherein the samples of inertial measurement values are obtained by the accelerometer. The accelerometer may be a piezoresistive accelerometer. Preferably, the accelerometer is a Micro Electro Mechanical Systems (MEMS) accelerometer. The accelerometer provides for counting the steps during walking. It is understood that the samples of inertial measurement values, or accelerometer data, may be, for example, as voltage or current signal.

It is understood that for the first step taken the average acceleration profile is the acceleration profile as such.

The system is configured such that the determination of the fall risk measure comprises:
- determining, during a first time interval, a change in width of at least one distribution of characterizing feature values,
- determining, during a second time interval, an average acceleration deviation value in relation to at least one average acceleration profile,
- determining, if the determined change in width of at least one distribution of characterizing feature values is a decreasing width and/or if the determined average acceleration deviation value in relation to at least one average acceleration profile is below a first threshold value, that the fall risk measure has a value that indicates an increased risk of falling.

In other words, such a configuration provides a reliable fall risk measure from one or two factors. The first factor being the change in width of at least one distribution of characterizing feature values, representing a measure of how many gaits the bipedal object uses and how different these gaits are with respect to each other. The second factor being the average acceleration deviation value in relation to at least one average acceleration profile, representing a measure of how the current gait used by the bipedal object fits with the repertoire of gaits used by the bipedal object.

A deviation in the first factor, in particular a decrease in width of at least one distribution of characterizing feature values, may be interpreted as the sum of gaits used by the bipedal object is decreasing. This in turn may be interpreted as a sign that the lifestyle of bipedal object has become less versatile, or that the bipedal object has begun using a more cautious way of walking even in situations that previously allowed a more relaxed way of walking.

A deviation in the second factor may be interpreted as a temporary problem, an obstacle or an injury that makes the bipedal object walk using a gait that does not fit with gaits used previously by the bipedal object.

It is to be noted that the fall risk measure that is determined is not necessarily to be considered as a percentage value of the risk of falling, but more of a determination of warnings at different levels. An administrator of the system may configure parameters such as time intervals and threshold values in order to set levels of sensitivity to the various deviations, i.e. how sensitive the system is to the magnitude of changes in the behavior of the bipedal object in order to generate an alarm indicating a risk of falling.

It is understood that the fall risk measure is a measure not related to any specific disease or a disfunction. Instead, the measure is a generic measure similar to a measure determining a weight or a length of the object. Ther fall risk measure itself does not disclose if the subject has a specific disease or disfunction, if any. Instead, it is understood that the fall risk measure is seen as a value on which further investigations may be based, or proposed. Further investigations may for example relate to troubleshooting or blood analysis.

The system may be configured such that the determination of a respective distribution of each characterizing feature value comprises updating the respective distribution of each characterizing feature value with weighted values using an exponentially weighted moving average (EWMA) method. Similarly, the system may also or alternatively be configured such that the updating of a respective average acceleration profile comprises updating the respective average acceleration profile with weighted values using an EWMA method.

Such configurations have an advantageous effect in that they allow a minimization of computing and memory resources. That is, during the determination of the distribution of each characterizing feature value from the samples, the distribution is updated without the need for retaining the samples in storage. Similarly, during the updating of an average acceleration profile, there is no need for retaining the samples used for the updating.

An overall advantage of the present disclosure may be appreciated by noting that distributions of characterizing feature values are used (time dependent, without saving all history of all values) to characterize discrete walking styles (gaits) or situations associated with the bipedal object, and to use the width of such distributions to detect in real time motorically related difficulties, or changing living patterns (in cases where the bipedal object is a human being).

The system may be configured such that the determination that steps are taken by the bipedal object comprises determining that a step is taken when all of a plurality of conditions are satisfied, said conditions comprising:
- a norm of an acceleration vector is greater than an acceleration norm threshold value,
- an angular velocity with respect to a pitch axis is negative and less than an angular velocity threshold value, the pitch axis having a direction that is horizontal and perpendicular to a current direction of walking,
- an angle of rotation with respect to the pitch axis since a previous step was determined is greater than a first angle of rotation threshold value,
- the angle of rotation with respect to the pitch axis since the previous step was determined is within a second angle of rotation threshold value around the value zero, and
- a time interval lapsed since a previous step has been determined is between a first time interval value and a second time interval value.

Such a configuration has an advantageous effect in that it is possible to distinguish setting down of the left foot from setting down the right foot, which is an important key in analyzing different phases of a step. A further advantage is that it is possible to determine steps using very low threshold values, which allows use of the system in situations where a bipedal object walks with an extremely careful gait, as is typically the case when the bipedal object uses a walking support such as a walker. Also, such a configuration allows distinguishing between steps that follow a natural walking cycle, and steps that are of a more sporadic nature (for example, when a bipedal object such as a person is taking a step sideways when cooking), which means that such sporadic nature steps can be disregarded.

The system may be configured such that the determination of a value of a characterizing feature of a step comprises any of:
- determining a step duration value for a step taken by the bipedal object,
- determining a step length value for a step taken by the bipedal object,
- determining a force exerted on a surface by impact of an end of the leg of the bipedal object,
- determining a maximum downward acceleration value among the acceleration value samples during a step taken by the bipedal object, and
- determining a correlation value based on acceleration value samples of consecutive steps taken by the bipedal object. In such cases, the system may be configured such that the determination of a correlation value based on acceleration value samples of consecutive steps taken by the bipedal object comprises determining a mean value of relative differences between a plurality of corresponding sample values of two consecutive steps taken by the bipedal object.

It is understood that any of the step duration value for a step taken by the bipedal object, the step length value for a step taken by the bipedal object, the force exerted on a surface by impact of an end of the leg of the bipedal object, the maximum downward acceleration value among the acceleration value samples during a step taken by the bipedal object, and the correlation value based on acceleration value samples of consecutive steps taken by the bipedal object may be a characterizing feature.

Using step duration values and step length values has an advantage of enabling determination of an enhanced fall risk that is not only related to motorical problems of the bipedal object, but also related to external factors (stress, for example, when a person with a history of frequent falling moves faster than the person normally does and thus presses themselves). Using values of impact force and downward acceleration has an advantage of enabling determination of an enhanced fall risk that is related to poorly controlled gait, such as when a person stumbles forward, for example in a case when a person who normally uses a walking support moves without using the walking support.

Using correlation values based on acceleration value samples of consecutive steps has an advantage of enabling determination of an enhanced fall risk that is related to the fact that persons with severely impaired motorical skills typically lose the ability to walk in a relaxed manner, even on smooth surfaces. Use of such correlation values may also take into account smaller variations in the environment, for example taking into account that outdoor walking is typically more irregular than walking indoors.

The system may be configured to output the fall risk measure that represents a risk for the bipedal object to fall when walking. As exemplified in the following detailed description, such output may comprise the characterizing feature distributions themselves and their changes over time together with a value that flags an increased fall risk as determined based on the width (or change of width) of at least one distribution of characterizing feature values and based on at least one determined acceleration deviation value.

The system may be confined to a single device or distributed between separate entities that communicate between each other. In other words, in some embodiments, the system comprises a housing within which the system is housed, said housing being configured to be attached to the leg of the bipedal object. In other embodiments, the system comprising a first part within which at least sensor circuitry and processing and communication circuitry is arranged, said first part being configured to be attached to the leg of the bipedal object, said processing and communication circuitry being configured to communicate with further processing circuitry via a communication network.

The object of the present disclosure to overcome drawbacks related to prior art methods and arrangements for determining the risk of falling is achieved in a second aspect by a method according to claim 10.

In a third aspect there is provided a computer program comprising instructions which, when executed on at least one processor in a system, cause the system to carry out the method according to the second aspect.

In a fourth aspect there is provided a carrier, comprising the computer program of the third aspect, wherein the carrier is one of an electronic signal, an optical signal, a radio signal and a computer readable storage medium.

In one embodiment there is provided a method for determining that steps are taken by the bipedal object when three or more, or five, conditions are satisfied, selected from:
- a norm of an acceleration vector is greater than an acceleration norm threshold value,
- an angular velocity with respect to a pitch axis is negative and less than an angular velocity threshold value, said pitch axis having a direction that is horizontal and perpendicular to a current direction of walking,
- an angle of rotation with respect to said pitch axis since a previous step was determined is greater than a first angle of rotation threshold value,
- the angle of rotation with respect to said pitch axis since the previous step was determined is within a second angle of rotation threshold value around the value zero, and
- a time interval lapsed since a previous step has been determined is between a first time interval value and a second time interval value.

It has been found that the method according to the fifth aspect is advantageous for determining that steps are taken by the bipedal object in different situations. For example, the method may be used for determining steps in situations where a bipedal object walks with an extremely careful gait, or taking short steps, as is typically the case when the bipedal object uses a walking support such as a walker.

The method according to the fifth aspect may be used in the method of the second aspect for for determining that steps are taken by the bipedal object. Additionally, the system according to the first aspect may be further configured to determine that steps are taken by the bipedal object by the method according to the fifth aspect.

These further aspects provide the same effects and advantages as those described in connection with the first aspect.

### BRIEF DESCRIPTION OF THE DRAWINGS

Figure 1 is a schematically illustrated bipedal object,
figure 2a is a schematically illustrated block diagram of a system for determining a fall risk measure,
figure 2b is a schematically illustrated block diagram of a system for determining a fall risk measure,
figure 2c is a schematically illustrated block diagram of a carrier comprising a computer program,
figure 3 is a flow chart.

### DETAILED DESCRIPTION

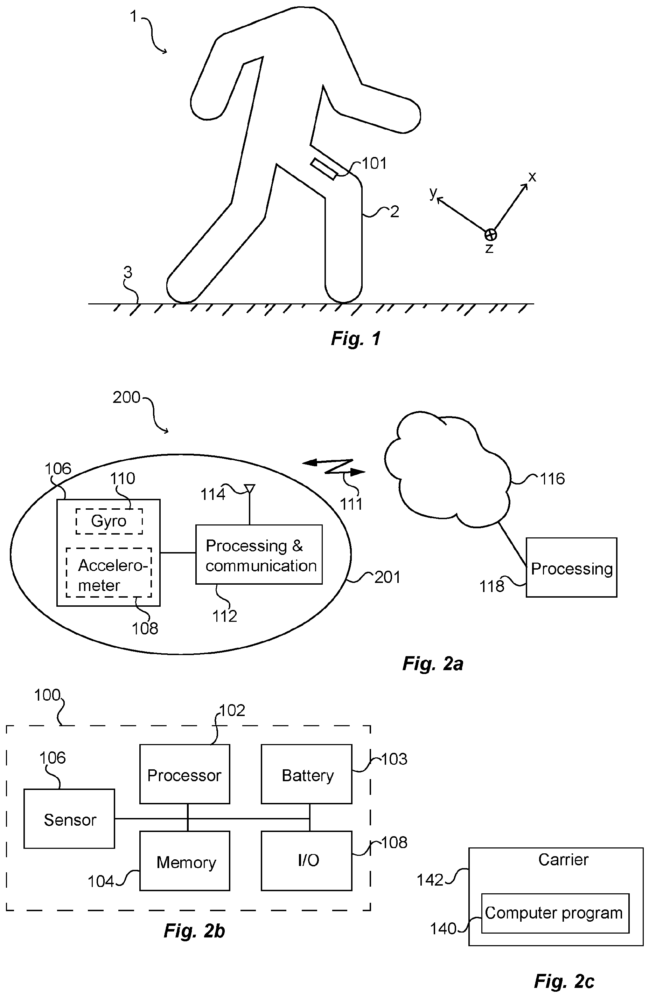

A bipedal object 1 walking on a surface 3 is illustrated very schematically in figure 1. The bipedal object 1 is a human being. Circuitry configured for determining fall risk measures is arranged within a housing 101 that is attached to a leg 2 of the bipedal object 1. Figure 1 also indicates an (x, y, z) system of coordinates used herein.

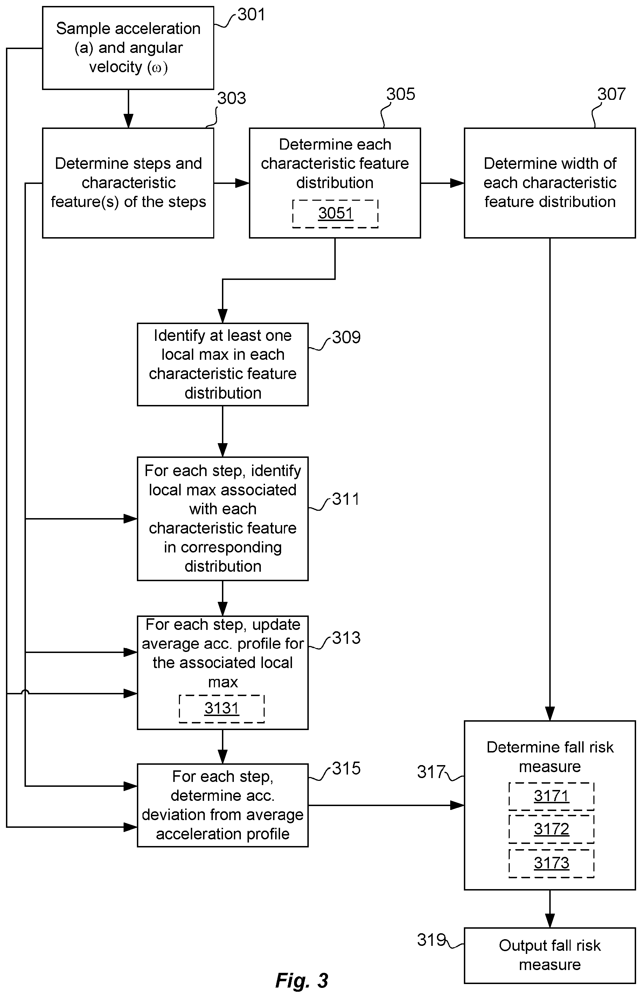

Turning now to figures 2a, 2b, 2c and figure 3 and with continued reference to figure 1, the various aspects of how the drawbacks associated with prior art fall risk measure determinations can be overcome.

As illustrated in figure 2a, a system 200 for determining, for a bipedal object 1 such as a human being or a robot, a fall risk measure that represents a risk for the bipedal object 1 to fall when walking, may comprise a plurality of functional entities: sensor circuitry 106 that may include an accelerometer 108 and a gyro 110, processing and communication circuitry 112 (that includes memory circuitry) that are connected to the sensor circuitry 106. The sensor circuitry 106 may thus be configured to sense acceleration and angular velocity in the x, y and z directions and provide a continuous stream of samples of instantaneous acceleration and angular velocity in the x, y and z directions, each sample associated with a time stamp obtained from a clock in the system 200. As the skilled person will realize, the x, y and z directions are defined in relation to the sensor circuitry 106. As exemplified in figure 1, the y direction is defined by the longitudinal direction of the housing 101, within which the sensor circuitry 106 is arranged, arranged laterally on the right side on the leg 2 of the bipedal object 1 and the x and z axes are mutually perpendicular and perpendicular to the y direction.

Such an arrangement of the sensor circuitry 106 on the leg 2 means that movement of the leg 2 during walking involves a rotation around the z-axis of the sensor circuitry 106, i.e. movement of the leg 2 is basically in a two-dimensional plane (x, y). It is of course possible to arrange the sensor circuitry 106 in relation to the leg 2 to allow for a more detailed three-dimensional representation of the steps taken by the bipedal object 1, where the sensor circuitry 106 rotates in all possible directions, but since walking by definition means that one foot is moved forward in front of another and the sensor circuitry 106 in the housing 101 is arranged laterally on the leg 2, it is possible to consider rotation only around the mediolateral axis (i.e. the z-axis, right to left in relation to the body of the bipedal object 1) in order to obtain the results as discussed herein. The z-axis may be denoted pitch axis, as used elsewhere herein.

The processing and communication circuitry 112 is configured to communicate, via a wireless interface 111, with further processing circuitry 118 connected in a communication network 116. That is, the system 200 may be divided into two or more processing parts - one part 201 located at the bipedal object 1 performing sensing and at least some processing of samples of data from the sensor circuitry 106, and a second part comprising the further processing circuitry 118 configured to perform at least part of the processing that will be described herein. For example, the first part 201 may be the housing 101 that houses the sensor circuitry 106 and the processing and communication circuitry 112, and the further processing circuitry 118 may form part of a so-called cloud computing service. Although not illustrated in figure 2a, the processing and communication circuitry 112 may comprise other input/output circuitry which may include communication circuitry, a display, a speaker, keys and/or any other suitable means for realizing a user interface. A user interface may also be configured such that access to the system 200 is made possible via the communication network 116, e.g. by means of a web interface, as the skilled person will realize. A battery may be included in the part 201 in order to provide appropriate electric power to the circuitry in the part 201.

Any details regarding the workings of the wireless interface 111 as such, the communication network 116 as such and the further processing circuitry 118 as such and any cloud computing service as such are outside the scope of the present disclosure.

As illustrated in figure 2b, a system 100 for determining, for a bipedal object 1 such as a human being or a robot, a fall risk measure that represents a risk for the bipedal object 1 to fall when walking, may comprise a plurality of functional entities: sensor circuitry 106 that may include an accelerometer and a gyro in the same way as described above in connection with the system 200 in figure 2a. The system 100 further comprises processing circuitry 102, memory 104, input/output circuitry 108 (which may include communication circuitry, a display, a speaker, keys and/or any other suitable means for realizing a user interface) and a battery 103, all of which are connected to the sensor circuitry 106. The system 100 may be housed within the housing 101 described above in connection with figure 1.

Figure 2c schematically illustrates a computer program 140 comprising instructions which, when executed on at least one processor 102, 118 in a system 100, 200, cause the system 100, 200 to operate as described herein. The carrier 142, comprising the computer program 140 may be an electronic signal, an optical signal, a radio signal, a computer readable storage medium, etc.

The system 100, 200 is thus configured to:
- obtain samples of inertial measurement values associated with movement of the leg 2 of the bipedal object 1, said samples of inertial measurement values comprising acceleration value samples and angular velocity value samples,
- determine, using the acceleration value samples and the angular velocity value samples, that steps are taken by the bipedal object 1 and a respective value of at least one characterizing feature of each step,
- determine a respective distribution of each characterizing feature value,
- determine a respective width of each distribution of characterizing feature values,
- identify, in each distribution of characterizing feature values, at least one local maximum,
- identify, for each step taken by the bipedal object 1, in each distribution of characterizing feature values, a local maximum closest to the value of the corresponding characterizing feature of the step,
- update, for each step taken by the bipedal object 1, a respective average acceleration profile with the acceleration value samples obtained for the step, where the respective average acceleration profile is associated with a respective identified local maximum closest to the value of the corresponding characterizing feature of the step,
- determine, for each step taken by the bipedal object 1, using the acceleration value samples obtained for the step, a respective acceleration deviation value in relation to at least one average acceleration profile, and
- determine, based on the width of at least one distribution of characterizing feature values and based on at least one determined acceleration deviation value, the fall risk measure that represents a risk for the bipedal object 1 to fall when walking.

Corresponding actions of a method of determining, for a bipedal object 1 such as a human being or a robot, fall risk measures that represent a risk for the bipedal object 1 to fall when walking, the method being performed by processing circuitry 102, 112, 118 in a system 100, 200, are illustrated in figure 3:

### Action 301

Samples are obtained of inertial measurement values associated with movement of a leg 2 of the bipedal object 1, said samples of inertial measurement values comprising acceleration value samples and angular velocity value samples.

### Action 303

A determination is made, using the acceleration value samples and the angular velocity value samples, that steps are taken by the bipedal object 1 of and a respective value of at least one characterizing feature of each step.

### Action 305

A determination is made of a respective distribution of each characterizing feature value.

### Action 307

A determination is made of a respective width of each distribution of characterizing feature values.

### Action 309

An identification is made, in each distribution of characterizing feature values, of at least one local maximum.

### Action 311

An identification is made, for each step taken by the bipedal object 1, in each distribution of characterizing feature values, of a local maximum closest to the value of the corresponding characterizing feature of the step.

### Action 313

An updating is made, for each step taken by the bipedal object 1, of a respective average acceleration profile with the acceleration value samples obtained for the step, where the respective average acceleration profile is associated with a respective identified local maximum closest to the value of the corresponding characterizing feature of the step.

### Action 315

A determination is made, for each step taken by the bipedal object 1, using the acceleration value samples obtained for the step, of a respective acceleration deviation value in relation to at least one average acceleration profile.

### Action 317

A determination is made, based on the width of at least one distribution of characterizing feature values and based on at least one determined acceleration deviation value, of the fall risk measure that represents a risk for the bipedal object 1 to fall when walking.

As mentioned above in relation to figures 2a and 2b, depending on how the system 100, 200 is configured, the processing may take place partly or wholly in circuitry 102 arranged in the housing 101 attached to the bipedal object 1 and in some cases also partly in the processing and communication circuitry 112 and in the further processing circuitry 118 connected via a communication network 116.

In some examples of how processing may be distributed, the sensor circuitry 106 that provides acceleration and angular velocity may be configured (corresponding to action 301) to provide output data at a given frequency, and also generate a "data ready" interrupt when a new sample is taken. The processing circuitry 102 in the housing 101 saves the generated data to a matrix in the memory 104 and applies logic (corresponding to action 303) to determine when a complete step cycle has taken place. The processing circuitry 102 in the housing 101 then carries out actions 305, 307, 309, 311, 313 and 315. Finally, the processing circuitry 102 in the housing 101 carries out action 307, 317 and, if applicable as will be described below, also action 319. Alternatively, the system 200 may be configured such that actions 307 and 317 are performed in the further processing circuitry 118 connected via a communication network 116. Configuration of the distribution of processing resources may depend on optimizations of data streams associated with the communication network 116 and access to the further processing circuitry 118, as well as what time frames the system 200 is configured to study. For example, in a scenario where long-term trends (e.g. several weeks or months) are to be analyzed, a system 200 as exemplified in figure 2a may be preferred since it may provide a practical way of accessing (for a user, a physiotherapist etc.) large amounts of data via, e.g., a web interface, as will be discussed below in connection with action 319.

As will be exemplified and described in more detail below, the characterizing features of steps may include one or more of step duration values for a step taken by the bipedal object 1, step length values for a step taken by the bipedal object 1, forces exerted on a surface 3 by impact of an end of the leg 2 of the bipedal object 1, maximum downward acceleration values among the acceleration value samples during a step taken by the bipedal object 1, and correlation values based on acceleration value samples of consecutive steps taken by the bipedal object 1.

The system 100, 200 is further configured such that the determination of the fall risk measure comprises:
- determining, during a first time interval, a change in width of at least one distribution of characterizing feature values,
- determining, during a second time interval, an average acceleration deviation value in relation to at least one average acceleration profile,
- determining, if the determined change in width of at least one distribution of characterizing feature values is a decreasing width and/or if the determined average acceleration deviation value in relation to at least one average acceleration profile is below a first threshold value, that the fall risk measure has a value that indicates an increased risk of falling.

Similarly, the method described may in some embodiments be configured such that the determination in action 317 of the fall risk measure comprises:

### Action 3171

A determination is made, during a first time interval, of a change in width of at least one distribution of characterizing feature values.

### Action 3172

A determination is made, during a second time interval, of an average acceleration deviation value in relation to at least one average acceleration profile.

### Action 3173

A determination is made, if the determined change in width of at least one distribution of characterizing feature values is a decreasing width and/or if the determined average acceleration deviation value in relation to at least one average acceleration profile is below a first threshold value, that the fall risk measure has a value that indicates an increased risk of falling.

It is to be noted that the fall risk measure that is determined by the system 100, 200 and the method, as exemplified in action 3173, may be considered as a determination of warnings at different levels. An administrator of the system 100, 200 may configure parameters such as the time intervals involved and the threshold values involved in order to set levels of sensitivity to the various deviations, i.e. how sensitive the system 100, 200 is to the magnitude of changes in the behavior of the bipedal object in order to generate a warning indicating a risk of falling.

Nevertheless, a warning may be generated on a linear combination of several or all changes and deviations (i.e. changes in distribution widths and acceleration deviation values). In such embodiments, it is important that the conditions (e.g. time intervals and thresholds) for the warnings are flexible, as this is used for monitoring a large range of users. For example, in case the bipedal object 1 is a human being it is easy to realize that conditions for a young person are different than those for an old person; a 35-year-old old probably has a much more animated movement pattern than an 80-90-year-old who only moves indoors using a walker.

Furthermore, although the determination that the fall risk measure has a value that indicates an increased risk of falling based on changes in distribution widths and/or acceleration deviation values may typically be a procedure that is performed during a substantial time period, e.g. days or weeks, it is also possible to generate a warning on a momentaneous time scale. For example, a warning may be determined immediately when it is determined that, e.g., the average acceleration deviation value in relation to at least one average acceleration profile is unusually large, i.e. above a second threshold value, which would indicate that the bipedal object 1 suddenly walks using a gait that it has not previously used and thereby representing an increased risk of falling.

The system 100, 200 may in some embodiments be configured such that the determination of a respective distribution of each characterizing feature value comprises updating the respective distribution of each characterizing feature value with weighted values using an exponentially weighted moving average (EWMA) method.

Similarly, the method described may in some embodiments be configured such that the determination in action 305 of a respective distribution of each characterizing feature value comprises:

### Action 3051

An updating is made of the respective distribution of each characterizing feature value with weighted values using an EWMA method.

In other words, with regard to how the system 100, 200 and the method, in action 305, may determine a respective distribution of each characterizing feature value, the following algorithm may be applied:
For each characterizing feature, a number of bins are defined within a reasonable range of the feature in question. For example, for step length, 50 bins are defined in the range of 0 cm - 150 cm, with a resolution of 3 cm. When an event (step) occurs, identification is made of within which bin the step falls (defined as bin *i*). The current value in each bin is defined as *Vⱼ* as: ${V}_{j} = α \times {V}_{j} for i \neq j$ ${V}_{j} = α \times {V}_{j} + \left(1-α\right) for i = j$ where α is a constant (α < 1) that determines the weighting of older events versus newer events. Mathematically, it is an exponentially weighted average filter that adds value 0 or value 1, depending on whether the current event falls within the given bin or not. The distribution that occurs in the bins corresponds to the distribution of events, but with a higher weight for newer events, with the advantage that there is no need for saving full information about all events that have taken place, thereby minimizing the memory space needed.

With regard to how the system 100, 200 and the method, in action 307, may determine a distribution width, it may be an arithmetic mean value of the number of bins between, e.g., the 10^{th} and 90^{th} percentile of the respective distribution.

With regard to how the system 100, 200 and the method, in action 309, may identify at least one local maximum in each distribution of characterizing feature values, a simple derivative analysis may be applied.

With regard to how the system 100, 200 and the method, in action 311, may identify a local maximum closest to the value of the corresponding characterizing feature of the step and how the system 100, 200 and the method, in action 313, may perform updating a respective average acceleration profile, the following algorithm may be utilized:
For each local maximum identified, an allocation is made of an array of accelerometer data. For example, 50 data points may be used each for three axes, although other number of points for each axis may be used depending on memory and processing resources in the system. That is, one or more 3x50 arrays of accelerometer data are allocated.

When an event (step) has been determined, a detection is made for each characterizing feature value which local maximum is the closest. Accelerometer data (i.e. the obtained acceleration value samples) for the step are interpolated into, e.g., 50 equidistant points, which are added to the corresponding point in the allocated array using an EMWA method. That is, each of the 50 data points from the new (down-sampled) step is added to the 3x50 matrix by means of an EWMA filter as follows:
Let the current value of this matrix for the x-axis be named *F_{Ax}, ₙ₋₁* and the x-axis acceleration from the latest step be named A_{x,n}. The updated filter value is then calculated as: ${F}_{Ax , n} = β \times {F}_{Ax , n - 1} + \left(1-β\right) \times {A}_{x , n}$

This is repeated for all three axes, and for each of the 50 samples in the down-sampled step. The result is a description of a typical step, which is simultaneously dependent on historical data and capable of changing with new data, with the advantage that there is no need for saving full information about all steps, thereby minimizing the memory space needed.

In other words, the system 100, 200 may in some embodiments be configured such that the updating of a respective average acceleration profile comprises updating the respective average acceleration profile with weighted values using an EWMA method.

Similarly, the method described may in some embodiments be configured such that the updating in action 313 of a respective average acceleration profile comprises:

### Action 3131

An updating is made of the respective average acceleration profile with weighted values using an EWMA method.

The result from such EWMA algorithm is several expected step profiles that depend on the values that the current step represents in terms of characterizing features such as step length, step duration, force exerted on a surface by impact, maximum downward acceleration, and acceleration value sample correlation with the previous step. Five deviation measures can easily be calculated between the current step and each step profile, and the average of these deviation measures may be used as a measure of how well the step matches or deviates from the expected acceleration profile.

The system 100, 200 may in some embodiments be configured such that the determination that steps are taken by the bipedal object 1 comprises determining that a step is taken when all of a plurality of conditions are satisfied, said conditions comprising:
- a norm of an acceleration vector is greater than an acceleration norm threshold value,
- an angular velocity with respect to a pitch axis is negative and less than an angular velocity threshold value, the pitch axis having a direction that is horizontal and perpendicular to a current direction of walking,
- an angle of rotation with respect to the pitch axis since a previous step was determined is greater than a first angle of rotation threshold value,
- the angle of rotation with respect to the pitch axis since the previous step was determined is within a second angle of rotation threshold value around the value zero, and
- a time interval lapsed since a previous step has been determined is between a first time interval value and a second time interval value.

Similarly, the method described may in some embodiments be configured such that the determination in action 303 that steps are taken by the bipedal object 1 comprises determining that a step is taken when all of a plurality of conditions are satisfied, said conditions comprising:
- a norm of an acceleration vector is greater than an acceleration norm threshold value,
- an angular velocity with respect to a pitch axis is negative and less than an angular velocity threshold value, the pitch axis having a direction that is horizontal and perpendicular to a current direction of walking,

- an angle of rotation with respect to the pitch axis since a previous step was determined is greater than a first angle of rotation threshold value,
- the angle of rotation with respect to the pitch axis since the previous step was determined is within a second angle of rotation threshold value around the value zero, and
- a time interval lapsed since a previous step has been determined is between a first time interval value and a second time interval value.

The norm *aₙ* of an acceleration vector may be determined by the simple formula: ${a}_{n} = \sqrt{{a}_{x}^{2} + {a}_{y}^{2} + {a}_{z}^{2}}$ where *aₓ, a_{y}* and *a_{z}* are acceleration value samples in the x, y, and z directions respectively.

As discussed above, the pitch axis is the mediolateral, z-axis, and the angular velocity with respect to the pitch axis may thus be determined more or less trivially by noting that the angular velocity samples provided by the sensor circuitry 106 relate to each of the x-, y- and z-axis. Consequently, the angular velocity samples relating to the pitch axis are the angular velocity samples of the z-axis.

The angle of rotation with respect to the pitch axis since a previous step was determined may be determined by integrating the angular velocity samples *ω_{z}* with respect to the z-axis during a time interval *Δt* and thereby obtaining the angle of rotation *ϕ_{z}* with respect to the pitch axis (z-axis) as: ${ϕ}_{z} = {ϕ}_{z} + Δt * {ω}_{z}$ where *ϕ*_{z} is re-set to zero each time a step has been determined.

The time interval lapsed since a previous step has been determined may be determined by determining differences between time stamps of subsequently determined steps, e.g. using time stamps of an internal clock in the system 100, 200.

As already indicated, the system 100, 200 may in some embodiments be configured such that the determination of a value of a characterizing feature of a step comprises any of:
- determining a step duration value for a step taken by the bipedal object 1,
- determining a step length value for a step taken by the bipedal object 1,
- determining a force exerted on a surface 3 by impact of an end of the leg 2 of the bipedal object 1,
- determining a maximum downward acceleration value among the acceleration value samples during a step taken by the bipedal object 1, and
- determining a correlation value based on acceleration value samples of consecutive steps taken by the bipedal object 1.

Similarly, the method described may in some embodiments be configured such that the determination in action 303 of a value of a characterizing feature of a step comprises any of:
- determining a step duration value for a step taken by the bipedal object 1,
- determining a step length value for a step taken by the bipedal object 1,
- determining a force exerted on a surface 3 by impact of an end of the leg 2 of the bipedal object 1,
- determining a maximum downward acceleration value among the acceleration value samples during a step taken by the bipedal object 1, and
- determining a correlation value based on acceleration value samples of consecutive steps taken by the bipedal object 1.

For example, the determination by the system 100, 200 and the method, as exemplified in action 303, of the values of the characterizing features may be realized as follows:
The step duration value for a step taken by the bipedal object 1 may be determined as a consequence of the determination that a step has been taken as described above. The step duration value is simply the time between each time a complete walking cycle is detected, i.e. the duration between two consecutive step determinations. Steps that have longer duration than a certain threshold (e.g. 2200 ms) may be considered "intermittent steps", and may therefore be discarded.

The step length value for a step taken by the bipedal object 1 may be determined by calculating the time integral of the angular velocity around the pitch axis, translating to a swept distance based on the leg 2 length of the bipedal object 1 and a typical conversion factor that accounts for the knee angle of the leg 2. Although such a calculation may not yield an exact value of the step length, e.g. due to uncertainties regarding knee angle, the step length value is correct within a few centimetres and thereby useful as a characterizing feature.

The force exerted on a surface 3 by impact of an end of the leg 2 of the bipedal object 1 may be determined by calculating the maximum acceleration norm from, e.g., 100 ms before to, e.g., 100 ms after the detected foot-down-event in accordance with the step detection logic described above.

The maximum downward acceleration value among the acceleration value samples during a step taken by the bipedal object 1 may be determined by calculating and tracking the minimum acceleration norm, calculated as described above, during the time between a detected step and the subsequent step.

A correlation value based on acceleration value samples of consecutive steps taken by the bipedal object 1 may be performed as follows: the correlation value between step n and step n-1 is defined as a mean value of the relative difference (measured as an absolute value, in percent) between each data point in step n, and the corresponding data point in step n-1, down sampled of up sampled such that the number of data points in step n-1 is equal to that of step n. If step n is composed by a number of samples, step n-1 is then down-/up-sampled to the same number of samples. For each of the three axes of the accelerometer, each sample from one step is compared to that of the previous, and a relative deviation is calculated for each sample. The purpose of this correlation value is to act as a measure of how regular the gait is. Typically, a user walking on uneven surfaces or in a crowded space will be displaying a less regular gait.

The system 100, 200 may in some embodiments be configured such that the determination of a correlation value based on acceleration value samples of consecutive steps taken by the bipedal object 1 comprises determining a mean value of relative differences between a plurality of corresponding sample values of two consecutive steps taken by the bipedal object 1.

Similarly, the method described may in some embodiments be configured such that the determination in action 303 of a correlation value based on acceleration value samples of consecutive steps taken by the bipedal object 1 comprises determining a mean value of relative differences between a plurality of corresponding sample values of two consecutive steps taken by the bipedal object 1.

The system 100, 200 may in some embodiments be configured to output the fall risk measure that represents a risk for the bipedal object 1 to fall when walking.

Similarly, the method described may in some embodiments comprise:

### Action 319

Outputting the fall risk measure that represents a risk for the bipedal object 1 to fall when walking.

With regard to how the system 100, 200 and the method, in action 319, may output the fall risk measure, it may be realized in various ways. The output is typically not in the form of a message: "you have X% risk of falling today", but rather in the form of values useable for, e.g., a physiotherapist who, by interpreting the values is made aware that a particular bipedal object such as a person has an increased fall risk compared to their normal state, and therefore may require extra attention/exercise. The actual values that, e.g., a physiotherapist may use for such an interpretation may comprise the characterizing feature distributions and their changes over time together with a value that flags an increased fall risk as determined in action 317 described above. Such output of values may typically take place via a web interface where the physiotherapist (or care assistant, etc.) can get an overview of a plurality of persons under observation, be warned if deviations are detected, and then click through to see exactly what the deviation consists of. The primary purpose is thus to flag high-fall risk persons, and communicate how they deviate, what the trend looks like, when it occurred, etc.

## Claims

1. A system (100, 200) for determining, for a bipedal object (1) being a human being, a fall risk measure that represents a risk for the bipedal object (1) to fall when walking, the system (100, 200) comprising circuitry (102, 104, 106, 112, 118) configured to:
- obtain samples of inertial measurement values associated with movement of a leg (2) of the bipedal object (1), said samples of inertial measurement values comprising acceleration value samples and angular velocity value samples,
- determine, using the acceleration value samples and the angular velocity value samples, that steps are taken by the bipedal object (1) and a respective value of at least one characterizing feature of each step,
- determine a respective distribution of each characterizing feature value,
- determine a respective width of each distribution of characterizing feature values,
- identify, in each distribution of characterizing feature values, at least one local maximum,
- identify, for each step taken by the bipedal object (1), in each distribution of characterizing feature values, a local maximum closest to the value of the corresponding characterizing feature of the step,
- update, for each step taken by the bipedal object (1), a respective average acceleration profile with the acceleration value samples obtained for the step, where the respective average acceleration profile is associated with a respective identified local maximum closest to the value of the corresponding characterizing feature of the step, wherein for the first step taken the average acceleration profile is the acceleration profile as such,
- determine, for each step taken by the bipedal object (1), using the acceleration value samples obtained for the step, a respective acceleration deviation value in relation to at least one average acceleration profile, and
- determine, based on the width of at least one distribution of characterizing feature values and based on at least one determined acceleration deviation value, the fall risk measure that represents a risk for the bipedal object (1) to fall when walking, wherein the system is configured such that the determination of the fall risk measure comprises:
- determining, during a first time interval, a change in width of at least one distribution of characterizing feature values,
- determining, during a second time interval, an average acceleration deviation value in relation to at least one average acceleration profile,
- determining, if the determined change in width of at least one distribution of characterizing feature values is a decreasing width and/or if the determined average acceleration deviation value in relation to at least one average acceleration profile is below a first threshold value, that the fall risk measure has a value that indicates an increased risk of falling.

2. The system (100, 200) of claim 1, configured such that the determination of a respective distribution of each characterizing feature value comprises:
- updating the respective distribution of each characterizing feature value with weighted values using an exponentially weighted moving average, EWMA, method.

3. The system (100, 200) of any of claims 1 to 2, configured such that the updating of a respective average acceleration profile comprises:
- updating the respective average acceleration profile with weighted values using an exponentially weighted moving average, EWMA, method.

4. The system (100, 200) of any of claims 1 to 3, configured such that the determination that steps are taken by the bipedal object (1) comprises determining that a step is taken when all of a plurality of conditions are satisfied, said conditions comprising:
- a norm of an acceleration vector is greater than an acceleration norm threshold value,
- an angular velocity with respect to a pitch axis is negative and less than an angular velocity threshold value, said pitch axis having a direction that is horizontal and perpendicular to a current direction of walking,
- an angle of rotation with respect to said pitch axis since a previous step was determined is greater than a first angle of rotation threshold value,
- the angle of rotation with respect to said pitch axis since the previous step was determined is within a second angle of rotation threshold value around the value zero, and
- a time interval lapsed since a previous step has been determined is between a first time interval value and a second time interval value.

5. The system (100, 200) of any of claims 1 to 5, configured such that the determination of a value of a characterizing feature of a step comprises any of:
- determining a step duration value for a step taken by the bipedal object (1),
- determining a step length value for a step taken by the bipedal object (1),
- determining a force exerted on a surface (3) by impact of an end of the leg (2) of the bipedal object (1),
- determining a maximum downward acceleration value among the acceleration value samples during a step taken by the bipedal object (1), and
- determining a correlation value based on acceleration value samples of consecutive steps taken by the bipedal object (1).

6. The system (100, 200) of claim 5, configured such that the determination of a correlation value based on acceleration value samples of consecutive steps taken by the bipedal object (1) comprises:
- determining a mean value of relative differences between a plurality of corresponding sample values of two consecutive steps taken by the bipedal object (1).

7. The system (100, 200) of any of claims 1 to 6, configured to:
- output the fall risk measure that represents a risk for the bipedal object (1) to fall when walking.

8. The system (100) of any of claims 1 to 7, comprising a housing (101) within which the system (100) is housed, said housing (101) being configured to be attached to the leg (2) of the bipedal object (1).

9. The system (100) of any of claims 1 to 7, comprising a first part (201) within which at least sensor circuitry (106) and processing and communication circuitry (112) is arranged, said first part (201) being configured to be attached to the leg (2) of the bipedal object (1), said processing and communication circuitry (112) being configured to communicate with further processing circuitry (118) via a communication network (116).

10. A method of determining, for a bipedal object (1) being a human being, fall risk measures that represent a risk for the bipedal object (1) to fall when walking, the method being performed by processing circuitry (102, 112, 118) in a system (100, 200), the method comprising:
- obtaining (301) samples of inertial measurement values associated with movement of a leg (2) of the bipedal object (1), said samples of inertial measurement values comprising acceleration value samples and angular velocity value samples,
- determining (303), using the acceleration value samples and the angular velocity value samples, that steps are taken by the bipedal object (1) and a respective value of at least one characterizing feature of each step,
- determining (305) a respective distribution of each characterizing feature value,
- determining (307) a respective width of each distribution of characterizing feature values,
- identifying (309), in each distribution of characterizing feature values, at least one local maximum,
- identifying (311), for each step taken by the bipedal object (1), in each distribution of characterizing feature values, a local maximum closest to the value of the corresponding characterizing feature of the step,
- updating (313), for each step taken by the bipedal object (1), a respective average acceleration profile with the acceleration value samples obtained for the step, where the respective average acceleration profile is associated with a respective identified local maximum closest to the value of the corresponding characterizing feature of the step, wherein for the first step taken the average acceleration profile is the acceleration profile as such,
- determining (315), for each step taken by the bipedal object (1), using the acceleration value samples obtained for the step, a respective acceleration deviation value in relation to at least one average acceleration profile, and
- determining (317), based on the width of at least one distribution of characterizing feature values and based on at least one determined acceleration deviation value, the fall risk measure that represents a risk for the bipedal object (1) to fall when walking, wherein the determination of the fall risk measure comprises:
- determining, during a first time interval, a change in width of at least one distribution of characterizing feature values,
- determining, during a second time interval, an average acceleration deviation value in relation to at least one average acceleration profile,
- determining, if the determined change in width of at least one distribution of characterizing feature values is a decreasing width and/or if the determined average acceleration deviation value in relation to at least one average acceleration profile is below a first threshold value, that the fall risk measure has a value that indicates an increased risk of falling.

11. A computer program (140) comprising instructions which, when executed on at least one processor (102, 118) in a system (100, 200), cause the system (100, 200) to carry out the method according to claim 10.

12. A carrier (142), comprising the computer program (140) of claim 11, wherein the carrier (142) is one of an electronic signal, an optical signal, a radio signal and a computer readable storage medium.

## Patentansprüche

1. System (100, 200) zum Bestimmen eines Sturzrisikomaßes für ein zweibeiniges Objekt (1), das ein Mensch ist, das ein Risiko für das zweibeinige Objekt (1), beim Gehen zu stürzen, darstellt, wobei das System (100, 200) eine Schaltung (102, 104, 106, 112, 118) umfasst, die konfiguriert ist zum:
- Erhalten von Abtastwerten von Trägheitsmesswerten, die mit der Bewegung eines Beins (2) des zweibeinigen Objekts (1) assoziiert sind, wobei die Abtastwerte von Trägheitsmesswerten Beschleunigungswert-Abtastwerte und Winkelgeschwindigkeitswert-Abtastwerte umfassen,
- Bestimmen, unter Verwendung der Beschleunigungswert-Abtastwerte und der Winkelgeschwindigkeitswert-Abtastwerte, dass Schritte von dem zweibeinigen Objekt (1) unternommen werden, und eines jeweiligen Wertes für mindestens ein charakterisierendes Merkmal jedes Schrittes,
- Bestimmen einer jeweiligen Verteilung jedes charakterisierenden Merkmalswertes,
- Bestimmen einer jeweiligen Breite jeder Verteilung von charakterisierenden Merkmalswerten,
- Identifizieren, in jeder Verteilung der charakterisierenden Merkmalswerte, mindestens eines lokalen Maximums,
- Identifizieren, für jeden von dem zweibeinigen Objekt (1) unternommenen Schritt in jeder Verteilung der charakterisierenden Merkmalswerte, eines lokalen Maximums, das dem Wert des entsprechenden charakterisierenden Merkmals des Schrittes am nächsten liegt,
- Aktualisieren, für jeden von dem zweibeinigen Objekt (1) unternommenen Schritt, eines jeweiligen durchschnittlichen Beschleunigungsprofils mit den für den Schritt erhaltenen Beschleunigungswert-Abtastwerten, wobei das jeweilige durchschnittliche Beschleunigungsprofil mit einem jeweiligen identifizierten lokalen Maximum assoziiert ist, das dem Wert des entsprechenden charakterisierenden Merkmals des Schrittes am nächsten liegt, wobei für den ersten unternommenen Schritt das durchschnittliche Beschleunigungsprofil das Beschleunigungsprofil als solches ist,
- Bestimmen, für jeden von dem zweibeinigen Objekt (1) unternommenen Schritt, unter Verwendung der für den Schritt erhaltenen Beschleunigungswert-Abtastwerte, eines jeweiligen Beschleunigungsabweichungswertes in Bezug auf mindestens ein durchschnittliches Beschleunigungsprofil, und
- Bestimmen, auf der Grundlage der Breite mindestens einer Verteilung von charakterisierenden Merkmalswerten und auf der Grundlage mindestens eines bestimmten Beschleunigungsabweichungswertes, des Sturzrisikomaßes, das das Risiko für das zweibeinige Objekt (1) darstellt, beim Gehen zu stürzen, wobei das System derart konfiguriert ist, dass die Bestimmung des Sturzrisikomaßes Folgendes umfasst:
- Bestimmen einer Änderung der Breite mindestens einer Verteilung von charakterisierenden Merkmalswerten im Laufe eines ersten Zeitintervalls,
- Bestimmen eines durchschnittlichen Beschleunigungsabweichungswertes in Bezug auf mindestens ein durchschnittliches Beschleunigungsprofil im Laufe eines zweiten Zeitintervalls,
- Bestimmen, wenn die bestimmte Änderung der Breite mindestens einer Verteilung von charakterisierenden Merkmalswerten eine abnehmende Breite ist und/oder wenn der bestimmte durchschnittliche Beschleunigungsabweichungswert in Bezug auf mindestens ein durchschnittliches Beschleunigungsprofil unter einem ersten Schwellenwert liegt, dass das Sturzrisikomaß einen Wert aufweist, der ein erhöhtes Risiko des Stürzens angibt.

2. System (100, 200) nach Anspruch 1, derart konfiguriert, dass die Bestimmung einer jeweiligen Verteilung jedes charakterisierenden Merkmalswertes Folgendes umfasst:
- Aktualisieren der jeweiligen Verteilung jedes charakterisierenden Merkmalswertes mit gewichteten Werten unter Verwendung eines Verfahrens des exponentiell gewichteten gleitenden Durchschnitts (EWMA).

3. System (100, 200) nach einem der Ansprüche 1 bis 2, derart konfiguriert, dass das Aktualisieren eines jeweiligen durchschnittlichen Beschleunigungsprofils Folgendes umfasst:
- Aktualisieren des jeweiligen durchschnittlichen Beschleunigungsprofils mit gewichteten Werten unter Verwendung eines Verfahrens des exponentiell gewichteten gleitenden Durchschnitts (EWMA).

4. System (100, 200) nach einem der Ansprüche 1 bis 3, derart konfiguriert, dass die Bestimmung, dass Schritte von dem zweibeinigen Objekt (1) unternommen werden, das Bestimmen umfasst, dass ein Schritt unternommen wird, wenn alle einer Vielzahl von Bedingungen erfüllt sind, wobei diese Bedingungen Folgendes umfassen:
- eine Norm eines Beschleunigungsvektors ist größer als ein Schwellenwert einer Beschleunigungsnorm,
- eine Winkelgeschwindigkeit bezüglich einer Nickachse ist negativ und kleiner als ein Schwellenwert der Winkelgeschwindigkeit, wobei die Nickachse eine Richtung aufweist, die horizontal und senkrecht zu einer aktuellen Gehrichtung verläuft,
- ein Drehwinkel bezüglich der Nickachse, seit ein vorheriger Schritt bestimmt wurde, ist größer als ein erster Schwellenwert des Drehwinkels,
- der Drehwinkel bezüglich der Nickachse, seit der vorherige Schritt bestimmt wurde, liegt innerhalb eines zweiten Schwellenwertes des Drehwinkels um den Wert Null herum, und
- ein Zeitintervall, das verstrichen ist, seit ein vorheriger Schritt bestimmt wurde, liegt zwischen einem ersten Zeitintervallwert und einem zweiten Zeitintervallwert.

5. System (100, 200) nach einem der Ansprüche 1 bis 5, derart konfiguriert, dass die Bestimmung eines Wertes eines charakterisierenden Merkmals eines Schrittes beliebiges von Folgendem umfasst:
- Bestimmen eines Schrittdauerwertes für einen Schritt, der von dem zweibeinigen Objekt (1) unternommen wurde,
- Bestimmen eines Schrittlängenwertes für einen Schritt, der von dem zweibeinigen Objekt (1) unternommen wurde,
- Bestimmen einer Kraft, die durch die Einwirkung eines Endes des Beins (2) des zweibeinigen Objekts (1) auf eine Oberfläche (3) ausgeübt wird,
- Bestimmen eines maximalen Abwärtsbeschleunigungswertes unter den Beschleunigungswert-Abtastwerten im Laufe eines Schrittes, der von dem zweibeinigen Objekt (1) unternommen wird, und
- Bestimmen eines Korrelationswertes auf der Grundlage von Beschleunigungswert-Abtastwerten aufeinanderfolgender Schritte, die von dem zweibeinigen Objekt (1) unternommen wurden.

6. System (100, 200) nach Anspruch 5, derart konfiguriert, dass die Bestimmung eines Korrelationswertes auf der Grundlage von Beschleunigungswert-Abtastwerten aufeinanderfolgender Schritte, die von dem zweibeinigen Objekt (1) unternommen wurden, Folgendes umfasst:
- Bestimmen eines Mittelwertes von relativen Differenzen zwischen einer Vielzahl entsprechender Werte von Abtastwerten von zwei aufeinanderfolgenden Schritten, die von dem zweibeinigen Objekt (1) unternommen wurden.

7. System (100, 200) nach einem der Ansprüche 1 bis 6, konfiguriert zum:
- Ausgeben des Sturzrisikomaßes, das ein Risiko für das zweibeinige Objekt (1) darstellt, beim Gehen zu stürzen.

8. System (100) nach einem der Ansprüche 1 bis 7, umfassend ein Gehäuse (101), innerhalb dessen das System (100) untergebracht ist, wobei das Gehäuse (101) konfiguriert ist, um an dem Bein (2) des zweibeinigen Objekts (1) befestigt zu werden.

9. System (100) nach einem der Ansprüche 1 bis 7, umfassend ein erstes Teil (201), innerhalb dessen mindestens eine Sensorschaltung (106) und eine Verarbeitungs- und Kommunikationsschaltung (112) angeordnet ist, wobei das erste Teil (201) konfiguriert ist, um an dem Bein (2) des zweibeinigen Objekts (1) befestigt zu werden, wobei die Verarbeitungs- und Kommunikationsschaltung (112) konfiguriert ist, um über ein Kommunikationsnetzwerk (116) mit weiterer Verarbeitungsschaltung (118) zu kommunizieren.

10. Verfahren zum Bestimmen von Sturzrisikomaßen für ein zweibeiniges Objekt (1), das ein Mensch ist, die ein Risiko für das zweibeinige Objekt (1) darstellen, beim Gehen zu stürzen, wobei das Verfahren durch Verarbeitungsschaltung (102, 112, 118) in einem System (100, 200) durchgeführt wird, wobei das Verfahren Folgendes umfasst:
- Erhalten (301) von Abtastwerten von Trägheitsmesswerten, die mit der Bewegung eines Beins (2) des zweibeinigen Objekts (1) assoziiert sind, wobei die Abtastwerte von Trägheitsmesswerten Beschleunigungswert-Abtastwerte und Winkelgeschwindigkeitswert-Abtastwerte umfassen,
- Bestimmen (303), unter Verwendung der Beschleunigungswert-Abtastwerte und der Winkelgeschwindigkeitswert-Abtastwerte, dass Schritte von dem zweibeinigen Objekt (1) unternommen werden, und eines jeweiligen Wertes für mindestens ein charakterisierendes Merkmal jedes Schrittes,
- Bestimmen (305) einer jeweiligen Verteilung jedes charakterisierenden Merkmalswertes,
- Bestimmen (307) einer jeweiligen Breite jeder Verteilung von charakterisierenden Merkmalswerten,
- Identifizieren (309), in jeder Verteilung der charakterisierenden Merkmalswerte, mindestens eines lokalen Maximums,
- Identifizieren (311), für jeden von dem zweibeinigen Objekt (1) unternommenen Schritt in jeder Verteilung der charakterisierenden Merkmalswerte, eines lokalen Maximums, das dem Wert des entsprechenden charakterisierenden Merkmals des Schrittes am nächsten liegt,
- Aktualisieren (313), für jeden von dem zweibeinigen Objekt (1) unternommenen Schritt, eines jeweiligen durchschnittlichen Beschleunigungsprofils mit den für den Schritt erhaltenen Beschleunigungswert-Abtastwerten, wobei das jeweilige durchschnittliche Beschleunigungsprofil mit einem jeweiligen identifizierten lokalen Maximum assoziiert ist, das dem Wert des entsprechenden charakterisierenden Merkmals des Schrittes am nächsten liegt, wobei für den ersten unternommenen Schritt das durchschnittliche Beschleunigungsprofil das Beschleunigungsprofil als solches ist,
- Bestimmen (315), für jeden von dem zweibeinigen Objekts (1) unternommenen Schritt, unter Verwendung der für den Schritt erhaltenen Beschleunigungswert-Abtastwerten, eines jeweiligen Beschleunigungsabweichungswertes in Bezug auf mindestens ein durchschnittliches Beschleunigungsprofil, und
- Bestimmen (317), auf der Grundlage der Breite mindestens einer Verteilung von charakterisierenden Merkmalswerten und auf der Grundlage mindestens eines bestimmten Beschleunigungsabweichungswertes, des Sturzrisikomaßes, das das Risiko für das zweibeinige Objekt (1) darstellt, beim Gehen zu stürzen, wobei die Bestimmung des Sturzrisikomaßes Folgendes umfasst:
- Bestimmen einer Änderung der Breite mindestens einer Verteilung von charakterisierenden Merkmalswerten im Laufe eines ersten Zeitintervalls,
- Bestimmen eines durchschnittlichen Beschleunigungsabweichungswertes in Bezug auf mindestens ein durchschnittliches Beschleunigungsprofil im Laufe eines zweiten Zeitintervalls,
- Bestimmen, wenn die bestimmte Änderung der Breite mindestens einer Verteilung von charakterisierenden Merkmalswerten eine abnehmende Breite ist und/oder wenn der bestimmte durchschnittliche Beschleunigungsabweichungswert in Bezug auf mindestens ein durchschnittliches Beschleunigungsprofil unter einem ersten Schwellenwert liegt, dass das Sturzrisikomaß einen Wert aufweist, der ein erhöhtes Risiko des Stürzens angibt.

11. Computerprogramm (140), umfassend Anweisungen, die, wenn auf mindestens einem Prozessor (102, 118) in einem System (100, 200) ausgeführt, das System (100, 200) veranlassen, das Verfahren nach Anspruch 10 zu realisieren.

12. Träger (142), umfassend das Computerprogramm (140) nach Anspruch 11, wobei der Träger (142) eines von einem elektronischen Signal, einem optischen Signal, einem Funksignal und einem computerlesbaren Speichermedium ist.

## Revendications

1. Système (100, 200) permettant de déterminer, pour un objet bipède (1) étant un être humain, une mesure du risque de chute qui représente le risque de chute de l'objet bipède (1) lors de la marche, le système (100, 200) comprenant des circuits (102, 104, 106, 112, 118) configurés pour :
- obtenir des échantillons de valeurs de mesure inertielle associées au mouvement d'une jambe (2) de l'objet bipède (1), lesdits échantillons de valeurs de mesure inertielle comprenant des échantillons de valeurs d'accélération et des échantillons de valeurs de vitesse angulaire,
- déterminer, à l'aide des échantillons de valeurs d'accélération et des échantillons de valeurs de vitesse angulaire, que des pas sont effectués par l'objet bipède (1) et une valeur respective d'au moins une caractéristique de chaque pas,
- déterminer une distribution respective de chaque valeur de caractéristique,
- déterminer une largeur respective pour chaque distribution des valeurs de caractéristiques,
- identifier, dans chaque distribution de valeurs de caractéristiques, au moins un maximum local,
- identifier, pour chaque pas effectué par l'objet bipède (1), dans chaque distribution de valeurs de caractéristiques, un maximum local le plus proche de la valeur de la caractéristique correspondante du pas,
- mettre à jour, pour chaque pas effectué par l'objet bipède (1), un profil d'accélération moyen respectif avec les échantillons de valeur d'accélération obtenus pour le pas, dans lequel le profil d'accélération moyen respectif est associé à un maximum local identifié respectif le plus proche de la valeur de la caractéristique correspondante du pas, dans lequel, pour le premier pas effectué, le profil d'accélération moyen est le profil d'accélération tel quel,
- déterminer, pour chaque pas effectué par l'objet bipède (1), à partir des échantillons de valeurs d'accélération obtenus pour le pas, une valeur d'écart d'accélération respective par rapport à au moins un profil d'accélération moyen, et
- déterminer, sur la base de la largeur d'au moins une distribution de valeurs de caractéristiques et sur la base d'au moins une valeur d'écart d'accélération déterminée, la mesure du risque de chute qui représente le risque de chute de l'objet bipède (1) lors de la marche, dans lequel le système est configuré de telle sorte que la détermination de la mesure du risque de chute comprenne :
- la détermination, au cours d'un premier intervalle de temps, d'une variation de largeur d'au moins une distribution de valeurs de caractéristiques,
- la détermination, au cours d'un deuxième intervalle de temps, d'une valeur d'écart d'accélération moyenne par rapport à au moins un profil d'accélération moyen,
- la détermination, si la variation déterminée de largeur d'au moins une distribution de valeurs de caractéristiques est une largeur décroissante et/ou si la valeur d'écart moyenne de l'accélération déterminée par rapport à au moins un profil d'accélération moyen est inférieure à une première valeur seuil, que la mesure du risque de chute présente une valeur qui indique un risque accru de chute.

2. Système (100, 200) selon la revendication 1, configuré de telle sorte que la détermination d'une distribution respective de chaque valeur de caractéristique comprenne :
- la mise à jour de la distribution respective de chaque valeur de caractéristique avec des valeurs pondérées à l'aide d'un procédé de moyenne mobile pondérée exponentielle (EWMA).

3. Système (100, 200) selon l'une quelconque des revendications 1 à 2, configuré de telle sorte que la mise à jour d'un profil d'accélération moyen respectif comprenne :
- la mise à jour du profil d'accélération moyen respectif avec des valeurs pondérées à l'aide d'un procédé de moyenne mobile pondérée exponentielle (EWMA).

4. Système (100, 200) selon l'une quelconque des revendications 1 à 3, configuré de telle sorte que la détermination de la réalisation de pas par l'objet bipède (1) consiste à déterminer qu'un pas est effectué lorsque toutes les conditions d'une pluralité de conditions sont satisfaites, lesdites conditions comprenant :
- une norme d'un vecteur d'accélération est supérieure à une valeur seuil de norme d'accélération,
- une vitesse angulaire par rapport à un axe de tangage est négative et inférieure à une valeur seuil de vitesse angulaire, ledit axe de tangage présentant une direction horizontale et perpendiculaire à la direction actuelle de marche,
- un angle de rotation par rapport audit axe de tangage depuis la détermination d'un pas précédent est supérieur à une première valeur seuil d'angle de rotation,
- l'angle de rotation par rapport à l'axe de tangage susmentionné depuis la détermination du pas précédent se situe dans une deuxième valeur seuil d'angle de rotation autour de la valeur zéro, et
- un intervalle de temps écoulé depuis la détermination d'un pas précédent se situe entre une première valeur d'intervalle de temps et une deuxième valeur d'intervalle de temps.

5. Système (100, 200) selon l'une quelconque des revendications 1 à 5, configuré de telle sorte que la détermination de la valeur d'une caractéristique d'un pas comprenne l'une quelconque parmi :
- la détermination d'une valeur de durée d'un pas effectué par l'objet bipède (1),
- la détermination d'une valeur de longueur de pas pour un pas effectué par l'objet bipède (1),
- la détermination d'une force exercée sur une surface (3) par l'impact d'une extrémité de la jambe (2) de l'objet bipède (1),
- la détermination d'une valeur d'accélération maximale vers le bas parmi les échantillons de valeurs d'accélération lors d'un pas effectué par l'objet bipède (1), et
- la détermination d'une valeur de corrélation basée sur des échantillons de valeurs d'accélération de pas consécutifs effectués par l'objet bipède (1).

6. Système (100, 200) selon la revendication 5, configuré de telle sorte que la détermination d'une valeur de corrélation basée sur des échantillons de valeurs d'accélération de pas consécutifs effectués par l'objet bipède (1) comprenne :
- la détermination d'une valeur moyenne des différences relatives entre une pluralité de valeurs d'échantillon correspondantes de deux pas consécutifs effectués par l'objet bipède (1).

7. Système (100, 200) selon l'une quelconque des revendications 1 à 6, configuré pour :
- produire la mesure du risque de chute qui représente un risque pour l'objet bipède (1) de tomber en marchant.

8. Système (100) selon l'une quelconque des revendications 1 à 7, comprenant un boîtier (101) dans lequel le système (100) est logé, ledit boîtier (101) étant configuré pour être fixé à la jambe (2) de l'objet bipède (1).

9. Système (100) selon l'une quelconque des revendications 1 à 7, comprenant une première partie (201) dans laquelle sont disposés au moins des circuits de capteurs (106) et des circuits de traitement et de communication (112), ladite première partie (201) étant configurée pour être fixée à la jambe (2) de l'objet bipède (1), lesdits circuits de traitement et de communication (112) étant configurés pour communiquer avec d'autres circuits de traitement (118) via un réseau de communication (116).

10. Procédé de détermination, pour un objet bipède (1) étant un être humain, de mesures de risque de chute qui représentent le risque de chute de cet objet bipède (1) lors de la marche, le procédé étant mis en œuvre par des circuits de traitement (102, 112, 118) dans un système (100, 200), le procédé comprenant :
- l'obtention (301) d'échantillons de valeurs de mesure inertielle associées au mouvement d'une jambe (2) de l'objet bipède (1), lesdits échantillons de valeurs de mesure inertielle comprenant des échantillons de valeurs d'accélération et des échantillons de valeurs de vitesse angulaire,
- la détermination (303), en utilisant les échantillons de valeur d'accélération et les échantillons de valeur de vitesse angulaire, que des pas sont effectués par l'objet bipède (1) et une valeur respective d'au moins une caractéristique de chaque pas,
- la détermination (305) d'une distribution respective de chaque valeur de caractéristique,
- la détermination (307) d'une largeur respective de chaque distribution de valeurs de caractéristiques,
- l'identification (309), dans chaque distribution de valeurs de caractéristiques, d'au moins un maximum local,
- l'identification (311), pour chaque pas effectué par l'objet bipède (1), dans chaque distribution de valeurs de caractéristiques, d'un maximum local le plus proche de la valeur de la caractéristique correspondante du pas,
- la mise à jour (313), pour chaque pas effectué par l'objet bipède (1), d'un profil d'accélération moyen respectif avec les échantillons de valeur d'accélération obtenus pour le pas, dans lequel le profil d'accélération moyen respectif est associé à un maximum local identifié respectif le plus proche de la valeur de la caractéristique correspondante du pas, dans lequel pour le premier pas effectué, le profil d'accélération moyen est le profil d'accélération tel quel,
- la détermination (315), pour chaque pas effectué par l'objet bipède (1), en utilisant les échantillons de valeurs d'accélération obtenus pour le pas, d'une valeur d'écart d'accélération respective par rapport à au moins un profil d'accélération moyen, et
- la détermination (317), sur la base de la largeur d'au moins une distribution de valeurs de caractéristiques et sur la base d'au moins une valeur d'écart d'accélération déterminée, de la mesure du risque de chute qui représente le risque de chute de l'objet bipède (1) lors de la marche, dans lequel la détermination de la mesure du risque de chute comprend :
- la détermination, au cours d'un premier intervalle de temps, d'une variation de largeur d'au moins une distribution de valeurs de caractéristiques,
- la détermination, au cours d'un deuxième intervalle de temps, d'une valeur d'écart d'accélération moyenne par rapport à au moins un profil d'accélération moyen,
- la détermination, si la variation déterminée de largeur d'au moins une distribution de valeurs de caractéristiques est une largeur décroissante et/ou si la valeur d'écart moyenne de l'accélération déterminée par rapport à au moins un profil d'accélération moyen est inférieure à une première valeur seuil, que la mesure du risque de chute présente une valeur qui indique un risque accru de chute.

11. Programme informatique (140) comprenant des instructions qui, lorsqu'elles sont exécutées sur au moins un processeur (102, 118) dans un système (100, 200), amènent le système (100, 200) à exécuter le procédé selon la revendication 10.

12. Support (142), comprenant le programme informatique (140) selon la revendication 11, dans lequel le support (142) est l'un parmi un signal électronique, un signal optique, un signal radio et un support de stockage lisible par ordinateur.
